# EUROPÄISCHE PATENTSCHRIFT

(11) **EP 2 075 033 B1**
(45) Veröffentlichungstag und Bekanntmachung des Hinweises auf die Patenterteilung: **01.07.2015**
(21) Anmeldenummer: 08169496.0
(22) Anmeldetag: 20.11.2008
(51) Int. Cl.: A61N 1/368, A61N 1/37, A61B 5/0452, A61N 1/362

(54) **Biventrikulärer Herzstimulator**
Biventricular heart stimulator
Stimulateur cardiaque biventriculaire

(30) Priorität: 20.12.2007 DE 102007062440; 12.04.2008 DE 102008018569
(43) Veröffentlichungstag der Anmeldung: 01.07.2009
(73) Patentinhaber: Biotronik CRM Patent AG, 6341 Baar (CH)
(72) Erfinder: Ismer, Bruno, 18059, Rostock (DE); Körber, Thomas, 18057, Rostock (DE); Voß, Wolfgang, 18198, Kritzmow (DE); von Knorre, Georg Heinrich, 18057, Rostock (DE); Riedel, Björn, 18057, Rostock (DE); Neumann, Andreas, 12437, Berlin (DE); Busch, Ulrich, 10318, Berlin (DE); Pilz, Jürgen, 12307, Berlin (DE); Brüggemann, Thomas, 12157, Berlin (DE); Peters, Ralf, 17498, Neuenkirchen (DE)
(74) Vertreter: Lindner-Vogt, Karin L.

(56) Entgegenhaltungen:
- EP-A- 1 393 774
- US-A- 5 265 601
- US-A1- 2002 128 688
- US-A1- 2006 235 481
- US-A1- 2007 078 489
- US-B1- 6 295 475
- US-B1- 7 225 017

## Beschreibung

Die Erfindung betrifft einen implantierbaren Herzstimulator für die kardiale Resynchronisationstherapie (CRT) eines Herzens. Der Herzstimulator kann ein Herzschrittmacher oder ein implantierbarer Kardioverter/Defibrillator (ICD) oder eine Kombination aus beidem sein, der in der Lage ist, einen oder beide Vorhöfe und eine oder beide Herzkammern zu stimulieren.

Typischerweise weist ein derartiger Herzstimulator für alle in seine Funktion einbezogenen Herzvorhöfe und Herzkammern jeweils eine Sensingeinheit und eine Stimulationseinheit auf. Diese Einheiten sind im Betrieb des Herzstimulators jeweils über Elektrodenleitungen mit an geeigneten Stellen im Herzen zu implantierenden Elektroden verbunden. Die Elektrodenleitung mit den Elektroden zum Erfassen elektrischer Potentiale im rechten Vorhof des Herzens sowie zur Abgabe rechtsatrialer Stimulationsimpulse sind als Beispiel typischerweise Bestandteil einer atrialen Elektrodenleitung. Die Elektroden zum Erfassen elektrischer Potentiale im rechten Ventrikel sowie zur Abgabe rechtsventrikulärer Stimulationsimpulse sind typischerweise an einer rechtsventrikulären Elektrodenleitung befestigt, deren distales Ende bis in den Apex des rechten Ventrikels ragt. Die Elektrodenleitung mit den Elektroden zum Erfassen elektrischer Potentiale im linken Ventrikel des Herzens sowie zur Abgabe linksventrikulärer Stimulationsimpulse sind typischerweise Bestandteil einer linksventrikulären Elektrodenleitung, die durch den Koronarsinus eines Herzens verlegt wird und daher auch als Koronarsinuselektrodenleitung bezeichnet wird. Alle Elektrodenleitungen sind typischerweise an ihrem proximalen Ende über normierte Steckverbindungen mit einem entsprechenden Herzstimulator verbunden.

Die typischen Stimulationsmodi eines Herzstimulators wie beispielsweise AAI, VVI, VDD oder DDD können als bekannt vorausgesetzt werden. Gleiches gilt für die Abgabe von Stimulationsimpulsen nur im Bedarfsfall (Demand-Schrittmacher), bei der die Abgabe eines Stimulationsimpulses an den Vorhof oder die jeweiligen Kammern eines Herzens dann unterbunden wird, wenn zuvor in einem entsprechenden Escape-Intervall eine jeweilige Eigenaktion (intrinsische Kontraktion) des Herzvorhofes oder der Herzkammer über eine zugeordnete Sensingeinheit des Herzstimulators erfasst wurde. Diese an sich bekannten Konzepte können auch bei dem hier beschriebenen Herzstimulator verwirklicht sein.

Für eine atrium-synchrone Stimulation insbesondere in einem biventrikulären DDD Stimulationsmodus ist ein möglichst gut an den individuellen Patienten angepasstes atrioventrikuläres Verzögerungsintervall (im Folgenden auch als atrioventrikuläre Verzögerungszeit oder als AV-Zeit bezeichnet) wünschenswert. Die atrioventrikuläre Verzögerungszeit beginnt mit einem atrialen Triggerereignis und endet mit einer Stimulation des Ventrikels. Das atriale Triggerereignis kann dabei je nach dem aktuellen Stimulationsmodus (VDD- oder DDD-Stimulation) entweder die über eine Vorhofelektrode elektrisch wahrgenommene natürliche Vorhofaktion oder ein Vorhofstimulus sein. Die atrioventrikulären Verzögerungszeiten werden somit durch zwei unterschiedliche Zeitintervalle bestimmt, wobei abhängig vom aktuellen Stimulationsmodus des Schrittmachers entweder diejenige für die DDD-Stimulation oder diejenige für die VDD-Stimulation wirksam ist.

Die interatriale Leitungszeit beginnt bei DDD-Stimulation mit dem rechtsatrialen Stimulus, bei VDD-Stimulation mit einem die rechtsatriale Kontraktion kennzeichnenden Signalmerkmal und endet in beiden Stimulationsmodi mit einem elektrischen Signalmerkmal, welches die linksatriale Kontraktion kennzeichnet.

Die Stimulation des Ventrikels (ventrikuläre Stimulation) führt in der Regel zu einer Kontraktion des stimulierten Ventrikels, also zu einem stimulierten ventrikulären Ereignis. Lediglich wenn der Ventrikel aufgrund einer entsprechend kurz zuvor erfolgen Kontraktion noch refraktär ist oder die Stärke des ventrikulären Stimulationsimpulses nicht ausreichend ist (unterschwellig ist), um wenigstens einige Zellen des ventrikulären Myokards zu depolarisieren, unterbleibt eine ventrikuläre Kontraktion nach ventrikulärer Stimulation. Ein ventrikulärer Stimulationsimpuls wird im übrigen im sogenannten Demand Modus des Herzstimulators gar nicht erst ausgelöst, wenn der Herzschrittmacher innerhalb eines von der atrioventrikulären Verzögerungszeit abhängigen ventrikulären Escape-Intervalls eine natürliche ventrikuläre Kontraktion (ein natürliches ventrikuläres Ereignis) erfasst. In jenem Fall wird die Abgabe des ventrikulären Stimulationsimpulses inhibiert. All dieses kann als bekannt vorausgesetzt werden.

Die Erfassung einer Kontraktion erfolgt üblicher Weise mit Hilfe von Elektroden, die elektrische Potentiale des Myokards im jeweiligen Herzareal (damit ist der jeweilige Vorhof oder die jeweilige Kammer eines Herzens gemeint) aufnehmen und durch eine jeweilige Sensingeinheit, die den jeweiligen zeitlichen Verlauf des aufgenommenen Potentials auswertet, und zwar im einfachsten Fall durch einen Schwellwertvergleich. Denn der Potentialverlauf zeigt im Falle von mit einer Kontraktion des Myokards im jeweiligen Herzareal einhergehen Depolarisationen des Myokards typische Signalspitzen.

Ein solcher Schwellwertvergleich zur Erkennung einer ventrikulären Kontraktion in einem mit einer ventrikulären Elektrode aufgenommenen Signal ist in US 2002/0128688A1 beschrieben.

Aus US 2006/0235481A1 eine Anordnung bekannt, bei der eine linksventrikuläre Elektrodenleitung eine zusätzliche, in der Mitte des Koronarsinus angeordnete Elektrode zur Erfassung linksatrialer Signale aufweist.

Der hier betroffene Herzstimulator ist ein biventrikulärer Herzstimulator, der grundsätzlich in der Lage ist, beide Ventrikel des Herzens ständig oder bei Bedarf zu stimulieren. Derartige biventrikuläre Herzstimulatoren dienen üblicherweise neben der Stimulation der beiden Ventrikel bei Bedarf auch der Stimulation des rechten Atriums und werden daher auch als 3-Kammer-Herzschrittmacher bezeichnet. Diese werden z. B. für eine an sich bekannte Resynchronisations-Therapie eingesetzt (CRT: cardiac resynchronization therapy).

Ziel der Erfindung ist es, einen biventrikulären Herzstimulator zu schaffen, der eine einfache Bestimmung der individuellen interatrialen Leitungszeiten für vorhofwahrnehmende und vorhofstimulierende atrioventrikuläre Stimulation erlaubt und damit die Voraussetzung für eine einfache Optimierung der AV-Zeiten für VDD- und DDD-Stimulation ohne zusätzlichen Aufwand bzw. Belastung für Arzt und Patient ermöglicht.

Erfindungsgemäß wird dieses Ziel durch einen Herzstimulator der eingangs genannten Art erreicht, der eine rechtsatriale Sensingeinheit aufweist, die mit einer rechtsatrialen Elektrode zur Aufnahme elektrischer Potentiale des Myokards eines rechten Atriums eines Herzen verbunden oder zu verbinden und ausgebildet ist, über die rechtsatriale Elektrode aufgenommene elektrische Signale zu verarbeiten und in dem über die rechtsatriale Elektrode aufgenommenen elektrischen Signal Signalmerkmale zu detektieren, die eine rechtsatriale Kontraktion kennzeichnen. Der Herzstimulator weist außerdem eine linksventrikuläre Sensingeinheit auf, die mit einer linksventrikulären Elektrode zur Aufnahme elektrischer Potentiale des Myokards eines linken Ventrikels eines Herzen verbunden oder zu verbinden und die ausgebildet ist, über die linksventrikuläre Elektrode aufgenommene elektrische Signale zu verarbeiten. Erfindungsgemäß ist die linksventrikuläre Sensingeinheit ausgebildet, in dem über die linksventrikuläre Elektrode aufgenommenen elektrischen Signal Signalmerkmale einer linksatrialen Depolarisation zu detektieren, welche eine linksatriale Kontraktion kennzeichnen. Darüber hinaus wird die linksventrikuläre Sensingeinheit in der Regel auch dazu ausgebildet sein, in üblicher Weise solche Signalmerkmale zu detektieren, welche eine linksventrikuläre Depolarisation und damit einer Kontraktion des linken Ventrikels kennzeichnen.

Die Erfindung bringt den Vorteil mit sich, dass für Erfassen des Auftretens linksatrialer Ereignisse keine zusätzlichen Elektrodenleitungen erforderlich sind, sondern lediglich die bei üblichen biventrikulären Herzstimulatoren ohnehin Vorhandenen, insbesondere die linksventrikuläre Elektrodenleitung. Die Erfindung beschreibt somit insbesondere auch eine einfache Möglichkeit, die AV-Zeiten zu optimieren, ohne dass der Implantationsaufwand erhöht wird. Die Elektrodenleitungen und das jeweils verwendete Implantat bleiben unverändert, lediglich die Auswertung der Sense-Signale im Herzstimulator wird verändert. Dies stellt einen großen Vorteil gegenüber z.B. der aus US 6,295,475 bekannten Anordnung dar.

Vorzugsweise sind die rechtsatriale Sensingeinheit und die linksventrikuläre Sensingeinheit mit einer Erfassungseinheit des Herzstimulators verbunden, die ihrerseits ausgebildet ist, die Dauer interatrialer Leitungszeiten durch Bestimmen der Zeitdifferenz zwischen dem Auftreten eines, abhängig von der Betriebsart (VDD- oder DDD-Stimulation) entweder einen rechtsatrialen Stimulus oder eine rechtsatriale Kontraktion kennzeichnenden Signalmerkmals und dem Auftreten einer diesen Ereignissen jeweils zuzuordnenden linksatrialen Kontraktion kennzeichnenden Signalmerkmals automatisch oder manuell zu bestimmen.

Die Erfassungseinheit kann dabei auch Bestandteil eines Programmiergerätes sein, zu dem die auszuwertenden Signale telemetrisch übertragen werden. Sie kann auch aus einer Kombination von Funktionselementen im Herzschrittmacher und im Programmiergerät bestehen.

Die Erfassungseinheit ist vorzugsweise weiter ausgebildet, anhand der interatrialen Leitungszeiten und ggf. weiterer Eingangsgrößen die atrioventrikulären Verzögerungsintervalle für vorhofwahrnehmende und vorhofstimulierende atrioventrikulärer Stimulation zu bestimmen. Dies kann beispielsweise durch Addition eines empirisch gefundenen Zeitintervalls oder auf die aus US 7,248,925 bekannte Weise geschehen.

Erfindungsgemäß ist die linksventrikuläre Sensingeinheit weiterhin ausgebildet, sowohl die linksventrikuläre als auch die linksatriale Kontraktion kennzeichnende Merkmale zu erfassen und voneinander zu unterscheiden.

Dies erfolgt anhand eines doppelten Schwellwertvergleiches mit zwei unterschiedlichen Schwellwerten. Wenn ein Spitzenwert im zeitlichen Verlauf des linksventrikulären Signals gleichzeitig einen ersten, niedrigeren und einen zweiten höheren von zwei Schwellwerten überschreitet, erfasst die linksventrikuläre Sensingeinheit ein linksventrikuläres Ereignis und gibt ein entsprechendes Ausgangssignal aus. Überschreitet der Spitzenwert im zeitlichen Verlauf des linksventrikulären Signals jedoch nur den ersten, niedrigeren und nicht den zweiten höheren der beiden Schwellwerte, detektiert die linksventrikuläre Sensingeinheit ein linksatriales Ereignis und gibt ein entsprechendes Ausgangssignal aus. Ein doppelter Schwellwertvergleich ist aus US 5,265,601 bekannt.

Da unter Umständen linksatriale Signale mit sehr kleiner Amplitude zu erwarten sind, ist die linksventrikuläre Sensingeinheit auch so ausgebildet, dass die Zeitpunkte der oben benannten Schwellwerte innerhalb eines Stimulationszyklusses erst nach Aufsummierung einer bestimmten Anzahl einzelner Herzaktionen im Sinne einer Signalmittelungstechnik bestimmt werden.

In jedem Falle erfolgt die Bestimmung der atrioventrikulären Verzögerungsintervalle für DDD- und VDD-Stimulation durch Vermessung von individuellen Zeitintervallen, die zwischen dem die rechtsatriale Stimulation oder Kontraktion kennzeichnenden Signalmerkmal und dem der linksatrialen Kontraktion zuzuordnenden Signalmerkmal, einem Schwellwert auftreten.

Die Vermessung der atrioventrikulären Verzögerungsintervalle kann dabei grundsätzlich sowohl manuell als auch automatisch im Herzstimulator oder im Programmiergerät oder in der Kombination beider erfolgen.

Der implantierbare Herzstimulator ist vorzugsweise ein Herzschrittmacher oder ein Cardioverter/Defibrillator.

Weitere vorteilhafte Ausgestaltungen ergeben sich durch Kombination der hier beschriebenen Merkmale untereinander und mit solchen Merkmalen, die aus dem Stand der Technik bekannt sind.

Die Erfindung soll nun anhand eines Ausführungsbeispiels unter Bezug auf die Figuren näher erläutert werden. Diese zeigen in
- Figur 1:: eine Darstellung eines biventrikulären Herzstimulators mit angeschlossenen, im Herzen angeordneten Elektroden;
- Figur 2:: ein schematisches Blockschaltbild eines Herzstimulators;
- Figur 3:: zeigt schematisch die über eine jeweilige rechtsatriale, rechtsventrikuläre und linksventrikuläre Elektrode aufgenommenen Sensingsignale.

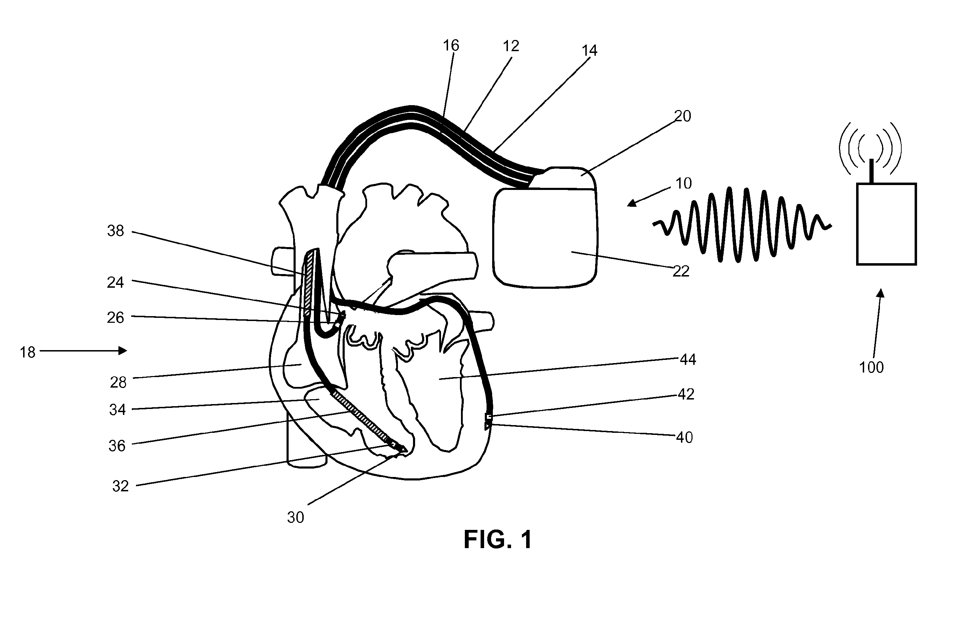

Figur 1 zeigt einen implantierbaren Herzstimulator 10 in Form eines Dreikammerherzschrittmachers/Kardioverter/Defibrillators mit daran angeschlossenen Elektrodenleitungen 12, 14 und 16 in Verbindung mit einem Herz 18. Außerdem ist ein externes Gerät 100 in der Nähe des implantierbaren Herzstimulators 10 dargestellt. Die Elektrodenleitungen 12, 14 und 16 sind über bekannte, standardisierte Steckverbindungen mit Kontaktbuchsen in einem Header (Anschlussgehäuse) 20 des Herzstimulators 10 elektrisch verbunden. Auf diese Weise sind die Elektrodenleitungen 12, 14 und 16 auch mit elektronischen Komponenten im Inneren eines hermetisch dichten Metallgehäuses 22 des Herzstimulators 10 verbunden. Diese Komponenten sind nachfolgend detaillierter schematisch dargestellt und bestimmen die erfindungsgemäße Funktionsweise des Herzstimulators 10.

Die Elektrodenleitung 12 ist eine rechtsatriale Elektrodenleitung und besitzt an ihrem distalen Ende eine atriale Spitzenelektrode RA Tip 24 sowie in kurzer Entfernung davon eine atriale Ringelektrode RA Ring 26, die beide im rechten Atrium 28 des Herzens 18 platziert sind.

Die Elektrodenleitung 14 ist eine rechtsventrikuläre Elektrodenleitung und besitzt an ihrem distalen Ende eine rechtsventrikuläre Spitzenelektrode RV Tip 30 und in unmittelbarer Nähe eine rechtsventrikuläre Ringelektrode RV Ring 32. Beide Elektroden sind im Apex des rechten Ventrikels 34 des Herzens 18 angeordnet.

Außerdem hat die rechtsventrikuläre Elektrodenleitung 14 noch eine rechtsventrikuläre Schockwendel RV Schock 36 als großflächige Elektrode zur Abgabe von Relationsschocks. Eine weitere Schockwendel 38 ist in der Vena Cava Superior angeordnet und wird deshalb nachfolgend auch als SVC-Schockelektrode bezeichnet.

Die Elektrodenleitung 16 ist eine linksventrikuläre Elektrodenleitung an deren distalem Ende eine linksventrikuläre Spitzenelektrode LV Tip 40 sowie in deren Nähe eine linksventrikuläre Ringelektrode LV Ring 42 angeordnet ist. Die linksventrikuläre Elektrodenleitung 16 wird vom rechten Atrium 28 des Herzens 18 aus über den Koronarsinus in eine von diesem abzweigende Lateralvene geführt und wird deswegen auch als Koronarsinuselektrodenleitung oder CS-Elektrodenleitung bezeichnet.

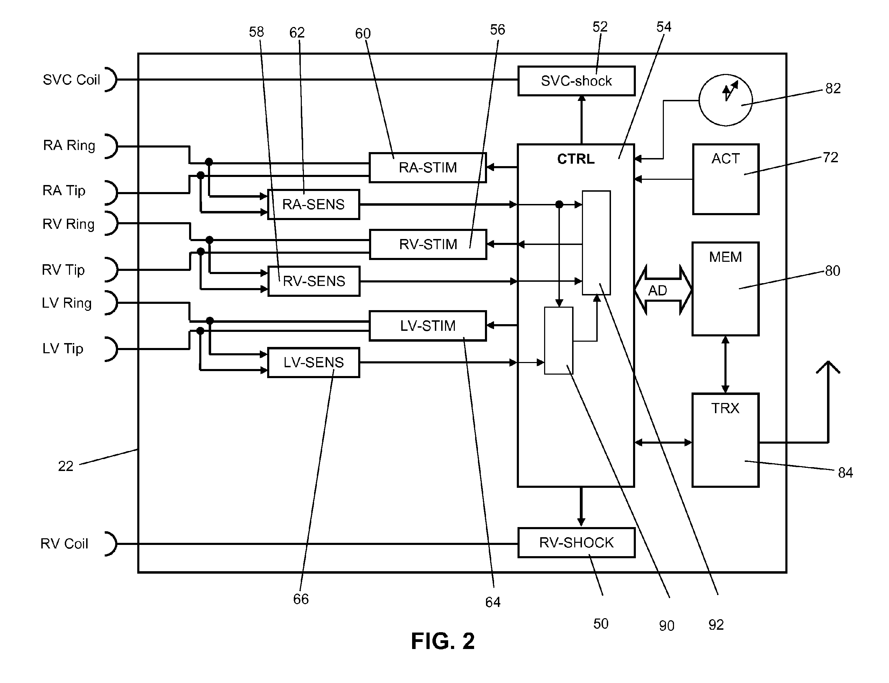

In Figur 2 sind die Hauptbestandteile des Herzstimulators 10 dargestellt. Auf der linken Seite sind die elektrischen Anschlüsse für die verschiedenen Elektroden 24, 26, 30, 32, 36, 38, 40 und 42 dargestellt. Die Schockelektroden 36 und 38 sind jeweils mit einem rechtsventrikulären Schockimpulsgenerator 50 bzw. SVC-Schockgenerator 52 verbunden. Beide Schockgeneratoren 50 und 52 sind mit einer Stimulationssteuereinheit 54 verbunden, die die beiden Schockimpulsgeneratoren 50 und 52 bei Bedarf zur Erzeugung und Abgabe eines Defibrillationsschocks ansteuert.

Der Anschluss für die rechtsventrikuläre Spitzenelektrode RV Tip sowie der Anschluss für die rechtsventrikuläre Ringelektrode RV Ring sind jeweils sowohl mit einer rechtsventrikulären Stimulationseinheit 56 als auch mit einer rechtsventrikulären Sensingeinheit 58 verbunden. Sowohl die rechtsventrikuläre Stimulationseinheit 56 als auch die rechtsventrikuläre Sensingeinheit 58 sind jeweils mit der Stimulationssteuereinheit 54 verbunden.

Die rechtsventrikuläre Stimulationseinheit 56 ist dazu ausgebildet, auf ein Ansteuersignal der Stimulationssteuereinheit 54 hin einen rechtsventrikulären Stimulationsimpuls zu erzeugen und über die Anschlüsse der rechtsventrikulären Ringelektrode RV Ring und der rechtsventrikulären Spitzenelektrode RV Tip abzugeben. Alternativ ist es auch möglich, dass das Gehäuse 22 des Herzstimulators 10 eine neutrale Elektrode bildet und die rechtsventrikuläre Stimulationseinheit 56 mit dem Anschluss für die rechtsventrikuläre Spitzenelektrode RV Tip und dem Gehäuse 22 als andere Elektrode zur Abgabe eines Stimulationsimpulses verbunden ist. Ein rechtsventrikulärer Stimulationsimpuls unterscheidet sich von einem Defibrillationsschock dadurch, dass der Stimulationsimpuls eine wesentlich geringere Impulsstärke besitzt, so dass er nicht wie ein Defibrillationsschock auf einen Schlag das vollständige Herzgewebe (Myokard) einer Herzkammer erregt, sondern nur die Herzmuskelzellen in unmittelbarer Umgebung der rechtsventrikulären Spitzenelektrode RV Tip 30. Diese Erregung breitet sich dann durch natürliche Reizleitung über den gesamten rechten Ventrikel 34 weiter aus und sorgt so für eine stimulierte Kontraktion des rechten Ventrikels 34.

Die rechtsventrikuläre Sensingeinheit 58 ist dazu ausgebildet, an dem Anschluss für die rechtsventrikuläre Ringelektrode RV Ring und die rechtsventrikuläre Tipelektrode RV Tip anliegende elektrische Potentiale zunächst durch einen Eingangsverstärker zu verstärken und zu filtern. Weiterhin ist die rechtsventrikuläre Sensingeinheit 58 ausgebildet, den Verlauf der an ihren Eingängen anliegenden elektrischen Signale derart auszuwerten, dass die rechtsventrikuläre Sensingeinheit 58 selbsttätig eine natürliche (intrinsische), d. h. selbsttätige Kontraktion des rechten Ventrikels 34 detektiert. Dies kann beispielsweise dadurch geschehen, dass der Verlauf des an den Eingängen der rechtsventrikulären Sensingeinheit 58 anliegenden Signals mit einem Schwellwert verglichen wird. Typischerweise ist die größte Amplitude des Signals in Form der sogenannten R-Zacke kennzeichnend für eine natürliche Kontraktion des rechten Ventrikels 34, die durch Schwellwertvergleich detektiert werden kann. Die rechtsventrikuläre Sensingeinheit 58 gibt daraufhin ein entsprechendes, eine natürliche Kontraktion des rechten Ventrikels 34 anzeigendes Ausgangssignal an die Stimulationssteuereinheit 54 aus.

In analoger Weise sind der Anschluss für die rechtsatriale Spitzenelektrode RA Tip und der Anschluss für die rechtsatriale Ringelektrode RA Ring sowohl mit einer rechtsatrialen Stimulationseinheit 60 als auch mit einer rechtsatrialen Sensingeinheit 62 verbunden, die jeweils ihrerseits wiederum mit der Stimulationssteuereinheit 54 verbunden sind. Die rechtsatriale Stimulationseinheit 60 ist dazu ausgebildet, Stimulationsimpulse zu erzeugen, deren Stärke ausreicht, das rechtsatriale Myokard zu erregen. Die rechtsatrialen Stimulationsimpulse können dabei eine andere Impulsstärke besitzen, als die rechtsventrikulären Stimulationsimpulse. Die rechtsatriale Sensingeinheit 62 ist ausgebildet, aus dem Verlauf des an ihren Eingängen anliegenden Differenzsignals eine sogenannte P-Welle zu detektieren, die eine natürliche (intrinsische) Kontraktion des rechten Atriums 28 kennzeichnet. Detektiert die rechtsatriale Sensingeinheit 62 eine entsprechende P-Welle, erzeugt sie ein Ausgangssignal und gibt dieses an die Stimulationssteuereinheit 54 weiter, welches eine natürliche Kontraktion des rechten Atriums 28 kennzeichnet.

In gleicher Weise sind auch der Anschluss für die linksventrikuläre Spitzenelektrode LV Tip und der Anschluss für die linksventrikuläre Ringelektrode LV Ring mit einer linksventrikulären Stimulationseinheit 64 und einer linksventrikulären Sensingeinheit 66 verbunden. Die linksventrikuläre Stimulationseinheit 64 und die linksventrikuläre Sensingeinheit 66 sind ebenso mit der Stimulationssteuereinheit 54 verbunden. Beide funktionieren ähnlich wie die bereits beschriebenen Stimulationseinheiten 56 und 60 und Sensingeinheiten 58 und 62.

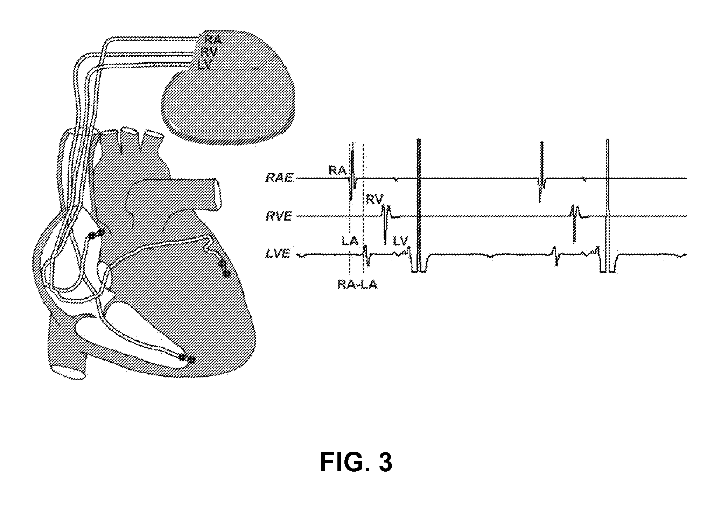

Im Unterschied zu bekannten linksventrikulären Sensingeinheiten ist die linksventrikuläre Sensingeinheit dazu ausgebildet, nicht nur eine linksventrikuläre Kontraktion kennzeichnende Signalmerkmale in dem aufgenommen elektrischen Signal zu detektieren, sondern auch solche Signalmerkmale, die eine linksatriale Kontraktion kennzeichnen, siehe Figur 3. Dementsprechend liefert die linksventrikuläre Sensingeinheit 66 unterschiedliche Ausgangssignale (Marker) für linksventrikuläre und linksatriale Ereignisse. Die Ausgangssignale (Marker) für linksatriale Ereignisse werden dabei für einmalige oder zyklische Bestimmung der Dauer interatrialen Leitungszeiten genutzt.

Hierzu ist die linksventrikuläre Sensingeinheit 66 so ausgeführt, dass sie einen doppelten Schwellwertvergleich mit zwei unterschiedlichen Schwellwerten durchführt. Wenn ein Spitzenwert im zeitliche Verlauf des linksventrikulären Signals gleichzeitig einen ersten, niedrigeren und einen zweiten höheren von zweiten Schwellwerten überschreitet, erfasst die linksventrikuläre Sensingeinheit ein linksventrikuläres Ereignis und gibt ein entsprechendes Ausgangssignal aus. Überschreitet der Spitzenwert im zeitlichen Verlauf des linksventrikulären Signals jedoch nur den ersten, niedrigeren und nicht den zweiten, höheren der beiden Schwellwerte, detektiert die linksventrikuläre Sensingeinheit ein linksatriales Ereignis und gibt ein entsprechendes Ausgangssignal aus.

Auch ist die linksventrikuläre Sensingeinheit so ausgebildet, dass die Zeitpunkte des Auftretens der oben benannten Schwellwerte innerhalb des Stimulationszyklus erst nach Aufsummierung einer bestimmten Anzahl einzelner Herzaktionen im Sinne einer Signalmittelungstechnik bestimmt werden.

Als weiterer Bestandteil des Herzstimulators 10 ist ein Beschleunigungssensor 72 mit der Stimulationssteuereinheit 54 verbunden und in das Gehäuse 22 des Herzstimulators 10 integriert. Der Beschleunigungssensor 72 ist dazu ausgebildet, ein von der körperlichen Aktivität eines Patienten abhängiges Bewegungssignal zu erfassen und ein entsprechendes, die körperliche Aktivität des Patienten anzeigendes erstes Akzelerometer-Ausgangssignal an die Stimulationssteuereinheit 54 auszugeben. Dies erlaubt es, dass die Stimulationssteuereinheit 54 das Timing der Stimulationsimpulse an den Bedarf des Patienten (hämodynamischen Bedarf) anpasst.

Weiterhin umfasst der Herzstimulator 10 eine Speichereinheit 80, die mit der Stimulationssteuereinheit 54 verbunden ist und es erlaubt, von der Stimulationssteuereinheit 54 erzeugte oder ausgewertete Signale zu speichern. Andererseits erlaubt es die Speichereinheit 80, Steuerprogramme für die Stimulationssteuereinheit 54 in veränderbarer Form zu speichern. Des Weiteren ist die Stimulationssteuereinheit 54 mit einem Zeitgeber 82 verbunden.

Die Speichereinheit 80 ist mit einer Telemetrieeinheit 84 verbunden, die es erlaubt, in der Speichereinheit 80 abgelegte Daten drahtlos an das externe Gerät 100 zu übertragen oder Programmierbefehle seitens des externen Geräts 100 zu dem Herzstimulator 10 zu übertragen und in der Speichereinheit 80 zu speichern.

Als Dreikammerherzstimulator/Kardioverter/Defibrillator ist der Herzstimulator 10 in der Lage, eine Stimulation des rechten Atriums 28, des rechten Ventrikels 34 und des linken Ventrikels 44 oder auch nur einer oder zweier dieser Herzkammern in an sich bekannter Weise durchzuführen. Dies schließt insbesondere die Stimulation einer jeweiligen Herzkammer im Demand-Modus ein, in der Stimulationsimpulse nur dann an die jeweilige Herzkammer abgegeben werden, falls in einem vorangehenden, jeweiligen Escape-Intervall seitens der jeweiligen Sensingeinheit keine intrinsische Kontraktion der jeweiligen Herzkammer erfasst wird. Damit ist der Herzstimulator 10 in der Lage, die bekannten rechtsventrikulären Stimulationsmodi wie VVI, VVD oder DDD durchzuführen.

Für das Timing der Stimulationsimpulse im biventrikulären Stimulationsmodus, in dem beide Ventrikel 34 und 44 eines Herzens 18 stimuliert werden, ist sowohl die interventrikulären Verzögerungszeit (VV-Intervall), d. h. diejenige Zeit, mit der ein rechter Stimulationsimpuls und ein linker Stimulationsimpuls (sofern sie im Demand-Modus nicht inhibiert werden) aufeinander folgen, als auch die für den jeweiligen Stimulationsmodus (VDD- oder DDD-Betrieb) notwendige atrioventrikuläre Verzögerungszeit (AV-Zeit) von Bedeutung. Diese wiederum wird idealerweise in Abhängigkeit von der jeweiligen individuellen, durch das Implantat bedingten interatrialen atrioventrikulären Leitungszeit eingestellt.

Um die implantatbedingten interatrialen Zeitintervalle für VDD- und DDD-Simulation zu bestimmen, besitzt die Stimulationssteuereinheit 54 erfindungsgemäß eine Erfassungseinheit 90. Diese kann mit den Sensingeinheiten beliebig auswählbarer Elektroden oder dem Schrittmachergehäuse verbunden werden. Sie lässt sich zum Beispiel vorzugsweise mit der rechtsatrialen Sensingeinheit 62 und der linksventrikulären Sensingeinheit 66 verbinden und ist ausgebildet, die Dauer der interatrialen Zeitintervalle zu bestimmen, indem die Erfassungseinheit 90 die Zeitdifferenz zwischen dem Auftreten eines einer rechtsatriale Kontraktion oder Stimulation kennzeichnenden Signalmerkmals und dem Auftreten eines einer linksatrialen, der rechtsatrialen Kontraktion zuzuordnenden Kontraktion kennzeichnenden Signalmerkmals bestimmt, d. h. die Zeitdifferenz zwischen zwei atrialen Ereignissen - nämlich (je nach Betriebsart) eines rechtsatrialen Stimulus oder einer rechtsatrialen Kontraktion und einer linksatrialen Kontraktion - eines Stimulationszyklusses.

Die Bestimmung der Dauer der interatrialen Zeitintervalle kann auch mit dem Programmiergerät erfolgen. Hierzu sind die entsprechenden Signalmerkmale enthaltenen Elektrogramme oder Marker telemetrisch vom Schrittmacher in das Programmiergerät zu übertragen. Die Erfassungseinheit ist dann Bestandteil des Programmiergerätes. Die Messungen erfolgen dann entweder automatisch im Programmiergerät oder manuell, indem die Elektrogramme oder Marker am Programmerbildschirm dargestellt, eingefroren und mittels Callippern vermessen werden.

Die Erfassungseinheit kann auch auf das externe Gerät 100 ausgelagert sein. Dann ist der Herzstimulator 10 ausgebildet, das über die linksventrikuläre Elektrode aufgenommene elektrische Signal beschreibende Daten mittels einer Telemetrieeinheit 84 telemetrisch auf das externe Gerät 100 zu übertragen.

Die Erfassungseinheit 90 ist mit einer AVD-Bestimmungseinheit 92 der Stimulationssteuereinheit 54 verbunden, die ein individuell abgestimmtes atrioventrikuläres Verzögerungsintervall (AV Intervall, AVD) auf Basis der jeweiligen interatrialen Leitungszeit bestimmt. Dies kann beispielsweise durch Addition eines empirisch gefundenen Zeitintervalls oder so erfolgen, wie dies in US 7,248,925 beschrieben ist.

Wie in Figur 2 beispielhaft dargestellt, ist die AVD-Bestimmungseinheit 92 außerdem ausgebildet, die rechtsventrikuläre Stimulationseinheit 56 zum Ende eines AV Intervalls auszulösen, falls sie nicht zuvor durch ein von der rechtsventrikulären Sensingeinheit 58 erfasstes rechtsventrikuläres Senseereignis zurückgesetzt wird, so dass ventrikuläre Stimulationen nur bei Bedarf abgegeben werden.

Figur 3 zeigt ein Schema einer Anordnung rechtsatrial (RA), rechtsventrikulär (RV) und linksventrikulär (LV) wahrnehmender und/oder stimulierender Schrittmacher- oder Defibrillator-Elektroden im Herzen (links) sowie das jeweilige davon elektrokardiographisch abgeleitete rechtsatriale (RAE), rechtsventrikuläre (RVE) und linksventrikuläre (LVE) Elektrogramm (rechts) als jeweiliges Sensingsignal. Die implantatbedingte interatriale Leitungszeit bei VDD-Stimulation ist in Figur 3 als Zeitintervall zwischen der rechtsatrialen und linksatrialen Deflektion messbar. Analog dazu ist die die interatrialen Leitungszeit bei DDD-Stimulation als Zeitintervall zwischen dem rechtsatrialen Stimulus und der linksatrialen Deflektion messbar.

**Bezugszeichenliste**

| **Bezugszeichen** | **Bedeutung** |
|---|---|
| 10 | Herzstimulator |
| 100 | externes Gerät |
| 12 | rechtsatriale Elektrodenleitung |
| 14 | rechtsventrikuläre Elektrodenleitung |
| 16 | linksventrikuläre Elektrodenleitung |
| 18 | Herz |
| 20 | Header (Anschlussgehäuse) |
| 22 | Gehäuse |
| 24 | atriale Spitzenelektrode RA Tip |
| 26 | atriale Ringelektrode RA Ring |
| 28 | rechtes Atrium |
| 30 | rechtsventrikuläre Spitzenelektrode RV Tip |
| 32 | rechtsventrikuläre Ringelektrode RV Ring |
| 34 | rechter Ventrikel |
| 36 | rechtsventrikuläre Schockwendel RV Schock |
| 38 | Schockwendel (SVG-Schockelektrode) |
| 40 | linksventrikuläre Spitzenelektrode LV Tip |
| 42 | linksventrikuläre Ringelektrode LV Ring |
| 44 | linker Ventrikel |
| 50 | rechtsventrikulärer Schockimpulsgenerator |
| 52 | SVC-Schockimpulsgenerator |
| 54 | Stimulationssteuereinheit |
| 56 | rechtsventrikuläre Stimulationseinheit |
| 58 | rechtsventrikuläre Sensingeinheit |
| 60 | rechtsatriale Stimulationseinheit |
| 62 | rechtsatriale Sensingeinheit |
| 64 | linksventrikuläre Stimulationseinheit |
| 66 | linksventrikuläre Sensingeinheit |
| 72 | Beschleunigungssensor |
| 80 | Speichereinheit |
| 82 | Zeitgeber |
| 84 | Telemetrieeinheit |
| 90 | Erfassungseinheit |
| 92 | AVD-Bestimmungseinheit |

## Patentansprüche

1. Implantierbarer Herzstimulator (10) mit einer rechtsatrialen Sensingeinheit (62), die mit einer rechtsatrialen Elektrode zur Aufnahme elektrischer Potentiale des Myokards eines rechten Atriums eines Herzen zu verbinden und ausgebildet ist, über die rechtsatriale Elektrode aufgenommene elektrische Signale zu verarbeiten und in dem über die rechtsatriale Elektrode aufgenommenen elektrischen Signal Signalmerkmale zu detektieren, die eine rechtsatriale Kontraktion kennzeichnen sowie mit einer linksventrikulären Sensingeinheit (66), die mit einer linksventrikulären, am Ende des Koronarsinus angeordneten, Elektrode zur Aufnahme elektrischer Potentiale des Myokards eines linken Ventrikels eines Herzen zu verbinden und ausgebildet ist, in dem über die linksventrikuläre Elektrode aufgenommenen elektrischen Signal Signalmerkmale zu detektieren, welche den Zeitpunkt der linksatrialen Kontraktion kennzeichnen,
**dadurch gekennzeichnet, dass**
die linksventrikuläre Sensingeinheit (66) ausgebildet ist, eine jeweilige linksventrikuläre Kontraktion und eine jeweilige linksatriale Kontraktion kennzeichnende Merkmale anhand eines doppelten Schwellwertvergleiches mit zwei unterschiedlichen Schwellwerten zu erfassen und voneinander zu unterscheiden, und das über die linksventrikuläre Elektrode aufgenommene elektrische Signal vor dem Schwellwertvergleich einer Signalmittelung über eine bestimmte Anzahl von Herzaktionen zu unterziehen.

2. Implantierbarer Herzstimulator (10) nach Anspruch 1, **dadurch gekennzeichnet, dass** die rechtsatriale Sensingeinheit (62) und die linksventrikuläre Sensingeinheit (66) mit einer Erfassungseinheit (90) verbunden sind, die ausgebildet ist, die Dauer interatrialer Leitungszeiten basierend auf der Bestimmung von Zeitdifferenzen festzulegen, deren Beginn bei Vorhofstimulation durch das Auftreten eines rechtsatrialen Stimulus und bei Vorhofwahrnehmung durch ein die rechtsatriale Kontraktion kennzeichnendes Signalmerkmal festgelegt ist und deren Ende durch das Auftreten eines die linksatriale Kontraktion kennzeichnenden Signalmerkmals charakterisiert ist.

3. Implantierbarer Herzstimulator (10) nach Anspruch 2, **gekennzeichnet durch** eine AVD-Bestimmungseinheit (92), die mit der Erfassungseinheit (90) verbunden und ausgebildet ist, anhand wenigstens der interatrialen Leitungszeit ein atrioventrikuläres Verzögerungsintervall zu bestimmen.

4. Herzstimulationssystem, das ein externes Gerät (100) und einen implantierbaren Herzstimulator (10) aufweist, wobei der implantierbare Herzstimulator (10) eine rechtsatriale Sensingeinheit (62), die mit einer rechtsatrialen Elektrode zur Aufnahme elektrischer Potentiale des Myokards eines rechten Atriums eines Herzens zu verbinden und ausgebildet ist, über die rechtsatriale Elektrode aufgenommene elektrische Signale zu verarbeiten und in dem über die rechtsatriale Elektrode aufgenommenen elektrischen Signal Signalmerkmale zu detektieren, die eine rechtsatriale Kontraktion kennzeichnen, eine linksventrikuläre Sensingeinheit (66), die mit einer linksventrikulären, am Ende des Koronarsinus angeordneten, Elektrode zur Aufnahme elektrischer Potentiale des Myokards eines linken Ventrikels eines Herzens zu verbinden und ausgebildet ist, über die linksventrikuläre Elektrode aufgenommene elektrische Signale zu verarbeiten, und eine Telemetrieeinheit (84) aufweist und ausgebildet ist, das über die linksventrikuläre Elektrode aufgenommene elektrische Signal beschreibende Daten telemetrisch auf das externe Gerät (100) zu übertragen,
**dadurch gekennzeichnet, dass** das externe Gerät (100) ausgebildet ist, eine jeweilige linksventrikuläre Kontraktion und eine jeweilige linksatriale Kontraktion kennzeichnende Merkmale anhand eines doppelten Schwellwertvergleiches mit zwei unterschiedlichen Schwellwerten zu erfassen und voneinander zu unterscheiden, und dass das externe Gerät und/oder die linksventrikuläre Sensingeinheit (66) ausgebildet ist, das über die linksventrikuläre Elektrode aufgenommene elektrische Signal vor dem Schwellwertvergleich einer Signalmittelung über eine bestimmte Anzahl von Herzaktionen zu unterziehen.

5. Herzstimulationssystem nach Anspruch 4 **dadurch gekennzeichnet, dass** das externe Gerät (100) ausgebildet ist, in dem über die linksventrikuläre Elektrode aufgenommenen elektrischen Signal Signalmerkmale darzustellen, welche den Zeitpunkt der linksatrialen Kontraktion kennzeichnen und deren Zeitabstand zu einem Signal oder Signalmerkmal, das den rechtsatrialen Stimulus oder eine rechtsatriale Kontraktion kennzeichnet, automatisch oder mittels Callipper manuell zu vermessen.

## Claims

1. An implantable cardiac stimulator (10) comprising a right-atrial sensing unit (62), which is intended to be connected to a right-atrial electrode for detecting electrical potentials of the myocardium of a right atrium of a heart and which is designed to process electrical signals detected via the right-atrial electrode and to detect signal features in the electrical signals detected via the right-atrial electrode that characterize a right-atrial contraction, and comprising a left-ventricular sensing unit (66), which is intended to be connected to a left-ventricular electrode disposed at the end of the coronary sinus for detecting electrical potentials of the myocardium of a left ventricle of a heart and which is designed to detect signal features in the electrical signal detected via the left-ventricular electrode that characterize the point in time of the left-atrial contraction,
**characterized in that**
the left-ventricular sensing unit (66) is designed to detect and differentiate features that characterize a respective left-ventricular contraction and a respective left-atrial contraction on the basis of a duplicate threshold-value comparison with two different threshold values, and to subject the electrical signal detected via the left-ventricular electrode before the threshold-value comparison to signal averaging over a certain number of cardiac activities.

2. The implantable cardiac stimulator (10) according to claim 1, **characterized in that** the right-atrial sensing unit (62) and the left-ventricular sensing unit (66) are connected to a detection unit (90), which is designed to determine the duration of interatrial conduction times on the basis of the determination of time differences, the beginning of which is defined, for atrial stimulation, by the occurrence of a right-atrial stimulus and, for atrial detection, by a signal feature characterizing the right-atrial contraction, and the end of which is **characterized by** the occurrence of a signal feature characterizing the left-atrial contraction.

3. The implantable cardiac stimulator (10) according to claim 2, **characterized by** an AVD determination unit (92), which is connected to the detection unit (90) and is designed to determine an atrioventricular delay interval on the basis of at least the interatrial conduction time.

4. A cardiac stimulation system, which comprises an external device (100) and an implantable cardiac stimulator (10), wherein the implantable cardiac stimulator (10) has a right-atrial sensing unit (62), which is intended to be connected to a right-atrial electrode for detecting electrical potentials of the myocardium of a right atrium of a heart and which is designed to process electrical signals detected via the right-atrial electrode and to detect signal features in the signal detected via the right-atrial electrode that characterize a right-atrial contraction, a left-ventricular sensing unit (66), which is intended to be connected to a left-ventricular electrode disposed at the end of the coronary sinus for detecting electrical potentials of the myocardium of a left ventricle of a heart and which is designed to process electrical signals detected via the left-ventricular electrode, and a telemetry unit (84), said implantable cardiac stimulator being designed to telemetrically transfer data, which describe the electrical signal detected via the left-ventricular electrode, to the external device (100),
**characterized in that** the external device (100) is designed to detect and differentiate features that characterize a respective left-ventricular contraction and a respective left-atrial contraction on the basis of a duplicate threshold-value comparison with two different threshold values, and the external device and/or the left-ventricular sensing unit (66) is designed to subject the electrical signal detected via the left-ventricular electrode before the threshold-value comparison to signal averaging over a certain number of cardiac activities.

5. The cardiac stimulation system according to claim 4, **characterized in that** the external device (100) is designed to depict signal features in the electrical signal detected via the left-ventricular electrode that characterize the point in time of the left-atrial contraction and to measure, automatically or manually by means of calipers, the time interval thereof from a signal or signal feature that characterizes the right-atrial stimulus or a right-atrial contraction.

## Revendications

1. Stimulateur cardiaque implantable (10) avec une unité de détection de l'oreillette droite (62) à relier à une électrode d'oreillette droite pour la réception de potentiels électriques du myocarde d'une oreillette droite d'un coeur, et conçue pour traiter des signaux électriques reçus par le biais de l'électrode d'oreillette droite et pour détecter, dans le signal électrique reçu par le biais de l'électrode d'oreillette droite, des caractéristiques de signal caractérisant une contraction de l'oreillette droite, et avec une unité de détection du ventricule gauche (66) à relier à une électrode de ventricule gauche, agencée à l'extrémité du sinus coronaire, pour la réception de potentiels électriques du myocarde d'un ventricule gauche d'un coeur, et conçue pour détecter, dans le signal électrique reçu par le biais de l'électrode de ventricule gauche, des caractéristiques de signal caractérisant le moment de la contraction du ventricule gauche,
**caractérisé en ce que**
l'unité de détection du ventricule gauche (66) est conçue pour détecter et différencier des caractéristiques caractérisant une contraction respective du ventricule gauche et une contraction respective de l'oreillette gauche, à l'aide d'une double comparaison de valeur seuil avec deux valeurs seuil différentes, et pour soumettre le signal électrique reçu par le biais de l'électrode de ventricule gauche à un calcul de moyenne sur un certain nombre d'actions cardiaques, avant la comparaison des valeurs seuil.

2. Stimulateur cardiaque implantable (10) selon la revendication 1, **caractérisé en ce que** l'unité de détection de l'oreillette droite (62) et l'unité de détection du ventricule gauche (66) sont reliées à une unité de détermination (90) conçue pour déterminer des durées de conduction inter-atriales sur la base de la détermination de différences temporelles, dont le début est défini par l'apparition d'un stimulus de l'oreillette droite en cas de stimulation auriculaire et par une caractéristique de signal caractérisant la contraction de l'oreillette droite en cas de détection auriculaire, et dont la fin est **caractérisée par** l'apparition d'une caractéristique de signal caractérisant la contraction de l'oreillette gauche.

3. Stimulateur cardiaque implantable (10) selon la revendication 2, **caractérisé par** une unité de détermination AVD (92) reliée à l'unité de détermination (90) et conçue pour déterminer un intervalle de temporisation atrio-ventriculaire, au moins à l'aide de la durée de conduction inter-atriale.

4. Système de stimulation cardiaque comprenant un appareil externe (100) et un stimulateur cardiaque implantable (10), dans lequel le stimulateur cardiaque implantable (10) comporte une unité de détection de l'oreillette droite (62) destinée à être reliée à une électrode d'oreillette droite pour la réception de potentiels électriques du myocarde d'une oreillette droite d'un coeur, et conçue pour traiter des signaux électriques reçus par le biais de l'électrode d'oreillette droite et pour détecter, dans le signal électrique reçu par le biais de l'électrode d'oreillette droite, des caractéristiques de signal caractérisant une contraction de l'oreillette droite, ainsi qu'une unité de détection du ventricule gauche (66) destinée à être reliée à une électrode de ventricule gauche, agencée à l'extrémité du sinus coronaire, pour la réception de potentiels électriques du myocarde d'un ventricule d'un coeur, et conçue pour traiter des signaux électriques reçus par le biais de l'électrode de ventricule gauche, et comportant une unité de télémétrie (84), tout en étant conçue pour transmettre par télémétrie des données décrivant le signal électrique reçu par le biais de l'électrode de ventricule gauche, à l'appareil externe (100),
**caractérisé en ce que** l'appareil externe (100) est conçu pour détecter et différencier des caractéristiques caractérisant une contraction respective du ventricule gauche et une contraction respective de l'oreillette gauche, à l'aide d'une double comparaison de valeurs seuil avec deux valeurs seuil différentes, et **en ce que** l'appareil externe et/ou l'unité de détection du ventricule gauche (66) est/sont conçu(s) pour soumettre le signal électrique reçu par le biais de l'électrode d'oreillette gauche à un calcul de moyenne sur un certain nombre d'actions cardiaques, avant la comparaison des valeurs seuil.

5. Système de stimulation cardiaque selon la revendication 4, **caractérisé en ce que** l'appareil externe (100) est conçu pour représenter, dans le signal électrique reçu par le biais de l'électrode de ventricule gauche, des caractéristiques de signal caractérisant le moment de la contraction de l'oreillette gauche, et pour mesurer leur écart temporel par rapport à un signal ou à une caractéristique de signal caractérisant le stimulus de l'oreillette droite ou une contraction de l'oreillette droite, automatiquement ou manuellement à l'aide d'un pied à coulisse.
